# EUROPEAN PATENT SPECIFICATION

(11) **EP 2 255 183 B1**
(45) Date of publication and mention of the grant of the patent: **02.10.2013**
(21) Application number: 09703561.2
(22) Date of filing: 26.01.2009
(51) Int. Cl.: G01N 33/48, G01N 35/02, G01N 35/10, B01L 3/00, G01N 35/00, H02N 15/00

(54) **ASSAY PREPARATION PLATES, FLUID ASSAY PREPARATION AND ANALYSIS SYSTEMS, AND METHODS FOR PREPARING AND ANALYZING ASSAYS**
TESTVORBEREITUNGSPLATTEN, FLUID-TESTVORBEREITUNGS- UND -ANALYSESYSTEME SOWIE VERFAHREN ZUR VORBEREITUNG UND ANALYSE VON TESTS
PLAQUES DE PRÉPARATION DE DOSAGES, SYSTÈMES D'ANALYSE ET DE PRÉPARATION DE DOSAGE DE FLUIDE ET PROCÉDÉS DE PRÉPARATION ET D'ANALYSE DE DOSAGE

(30) Priority: 25.01.2008 US 23671 P; 17.04.2008 US 45721 P
(43) Date of publication of application: 01.12.2010
(73) Proprietor: Luminex Corporation, Austin, TX 78727 (US)
(72) Inventor: SCHILFFARTH, Adam, Cedar Park, TX 78613 (US); DEICHER, William, Austin, TX 78729 (US); PEMPSELL, Paul, Bedford, TX 76022 (US)
(74) Representative: Lohr, Georg
(86) International application number: PCT/US2009/032022
(87) International publication number: WO 2009/094642

(56) References cited:
- EP-A1- 0 262 760
- US-A- 5 200 151
- US-A- 5 236 824
- US-A1- 2003 040 129
- US-A1- 2007 166 835
- US-B1- 6 858 440
- US-B2- 6 968 037

## Description

### BACKGROUND OF THE INVENTION

### 1. Field of the Invention

This invention generally relates to methods, systems, and devices for processing and analyzing assays and, more specifically, to methods, systems, and devices configured to allow assays to be processed with magnetic particles within an assay preparation plate at a fluid assay analysis system.

### 2. Description of the Related Art

The following descriptions and examples are not admitted to be prior art by virtue of their inclusion within this section.

Analysis of fluid assays is used for a variety of purposes, including but not limited to biological screenings and environmental assessments. In some cases, a fluid may be processed prior to being analyzed to remove matter which is not of interest or which may conflict with obtaining accurate analysis results. In addition or alternatively, a fluid may be processed prior to being analyzed to offer results of greater sensitivity and/or specificity. Moreover, a fluid may, in some embodiments, be processed prior to being analyzed to convert the fluid into a form that is compatible with a particular analysis method, such as into an assay which is particle-based. In any of such cases, the processing of fluid samples is generally conducted manually and, consequently, the benefit of the preparation of a particular assay-type and/or obtaining results of greater sensitivity and/or specificity may, in some cases, be jeopardized by the intrinsic variability of manual processes. Although efforts to automate the preparation of fluid assays have been attempted, such endeavors have met limited success due to difficulty in automating the removal of reagents used to process the sample as well as portions of the sample which are not of interest or which may conflict with obtaining accurate analysis results. Furthermore, most of such systems are relatively bulky and are further cost prohibitive for many companies and institutions due to their maintenance requirements and initial equipment costs.
EP 0 262 760 A1 discloses a test plate comprising a large number of openings, each supporting a cup. In the cup are magnetic beads to which antibodies are bound. Below the test plate is a rod, being reciprocated by a cam mechanism. On the rod a large number of magnets are positioned to be below the test cups. Thus, according to the motion of the magnets, the beads in the test cups will migrate.
US 5,236,824 discloses an in-situ magnetic immunoassay method. The system for applying the method comprises an assay plate receiving area, a pipette disposed above, a mechanism for moving the assay plate for aligning wells in the assay plate with the pipette. An examination chamber is coupled to the pipette for analyzing light being emitted or scattered by essay particles in the examination chamber.

### SUMMARY OF THE INVENTION

An embodiment of an assay preparation plate includes an array of wells, a magnet, and an actuator configured to move the magnet proximate and remote relative to one or more select wells of the array of wells.

An embodiment of a method for preparing and analyzing an assay includes injecting a sample for analysis into a sample well of an assay preparation plate of claim 1 and inserting the assay preparation plate into an assay plate receiving area of a fluid assay analysis system. The method further includes establishing a position of the assay preparation plate within the assay plate receiving area such that a particular well of the assay preparation plate is aligned with a pipette of the fluid assay analysis system and aspirating a fluidic material disposed within the particular well via the pipette. Moreover, the method includes moving the assay preparation plate within the assay plate receiving area such that a different well of the assay preparation plate is aligned with the pipette and dispensing the fluidic material into the different well. In general, the method may include repeating the steps of establishing, aspirating, moving, and dispensing to mix the sample with one or more reagents until preparation of an assay is complete. At least one series of the steps of establishing, aspirating, moving, and dispensing includes mixing the sample with a plurality of magnetic particles and, thereafter, immobilizing the plurality of magnetic particles in a well of the assay preparation plate. The method includes aspirating the assay from the assay preparation plate into an examination chamber of the fluid assay system via the pipette and a fluidic line coupled between the pipette and the examination chamber. Moreover, the method includes analyzing the assay within the examination chamber.

An embodiment of a fluid assay preparation and analysis system includes an assay preparation plate according to claim 1, an assay plate receiving area, a pipette disposed above the assay plate receiving area, and a magnet disposed below the assay plate receiving area in approximate alignment with the pipette. In addition, the fluid assay preparation and analysis system includes an actuator configured to move the magnet to and from a position proximate the assay plate receiving area and a mechanism for moving an assay plate disposed within the assay plate receiving area such that different wells of the assay plate are aligned with the pipette at different times. The fluid assay preparation and analysis system further includes an examination chamber coupled to the pipette via a fluidic line and an illumination system configured to illuminate the examination chamber. Moreover, the fluid assay preparation and analysis system includes a detection system configured to collect light emitted and/or scattered from assay particles introduced into the examination chamber via the pipette and the fluidic line. The detection system is further configured to generate signals representative of a degree of light gathered. The fluid assay preparation and analysis system further includes an examination system for analyzing the generated signals.

### BRIEF DESCRIPTION OF THE DRAWINGS

Other objects and advantages of the invention will become apparent upon reading the following detailed description and upon reference to the accompanying drawings in which:

Fig. 1 illustrates a perspective view of an exemplary fluid assay analysis system;

Fig. 2 illustrates a perspective view of an exemplary assay preparation plate;

Fig. 3 illustrates a perspective view of another exemplary assay preparation plate;

Fig. 4A illustrates a perspective view of the assay preparation plate depicted in Fig. 2 with its exterior casing removed;

Fig. 4B illustrates a top view of the assay preparation plate depicted in Fig. 4A;

Fig. 5A illustrates a partial schematic drawing of a fluid assay preparation and analysis system having a magnet actuator disposed below a pipette of the system;

Fig. 5B illustrates a partial schematic view of the fluid assay preparation and analysis system depicted in Fig. 5A in which the magnet actuator has moved a magnet in the vicinity of an assay plate receiving area interposed between the pipette and the magnet actuator; and

Fig. 6 illustrates a flow chart of an exemplary method for preparing and analyzing an assay.

### DETAILED DESCRIPTION OF THE PREFERRED EMBODIMENTS

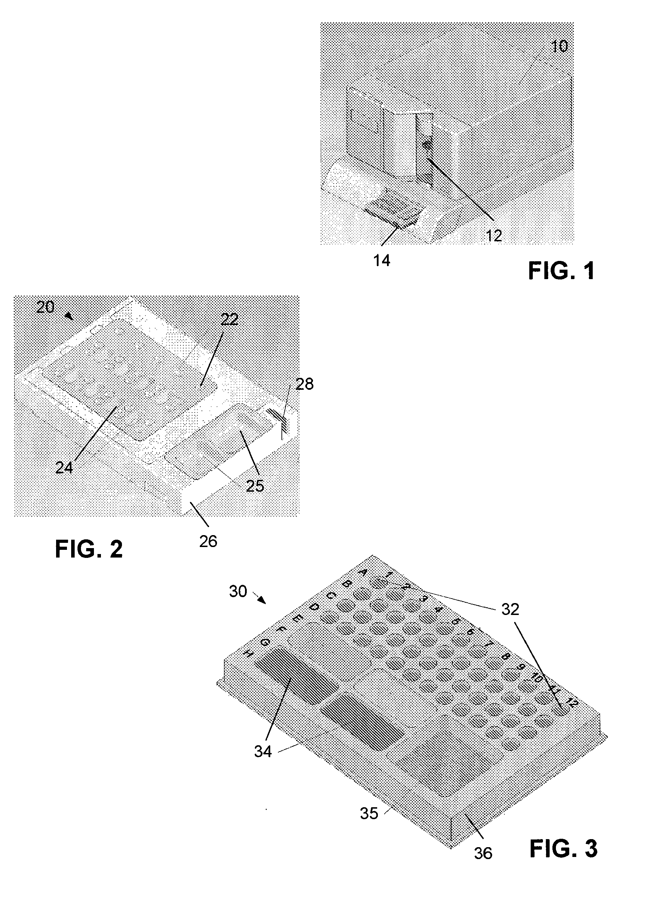

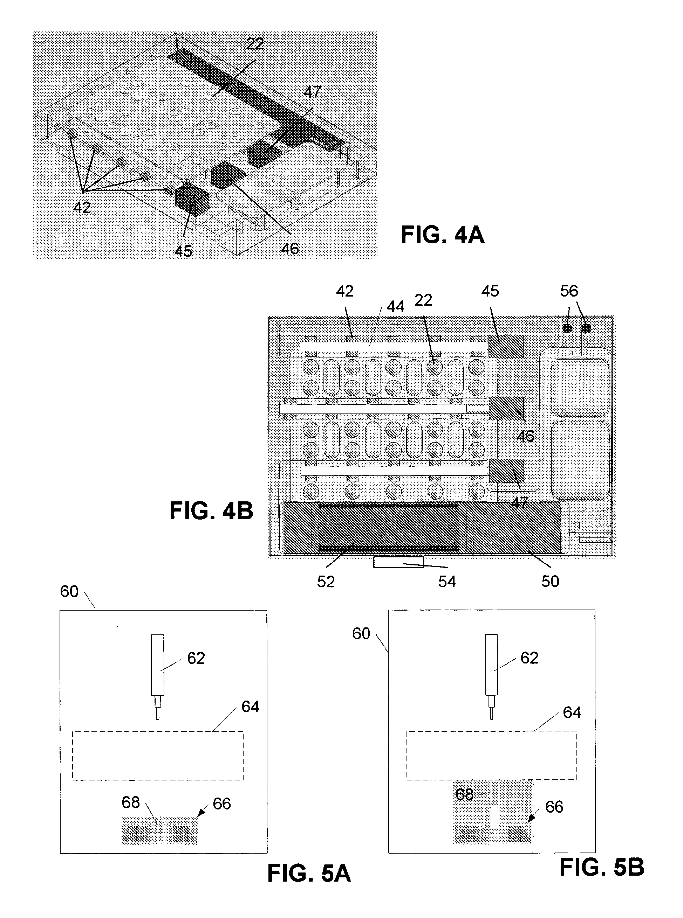

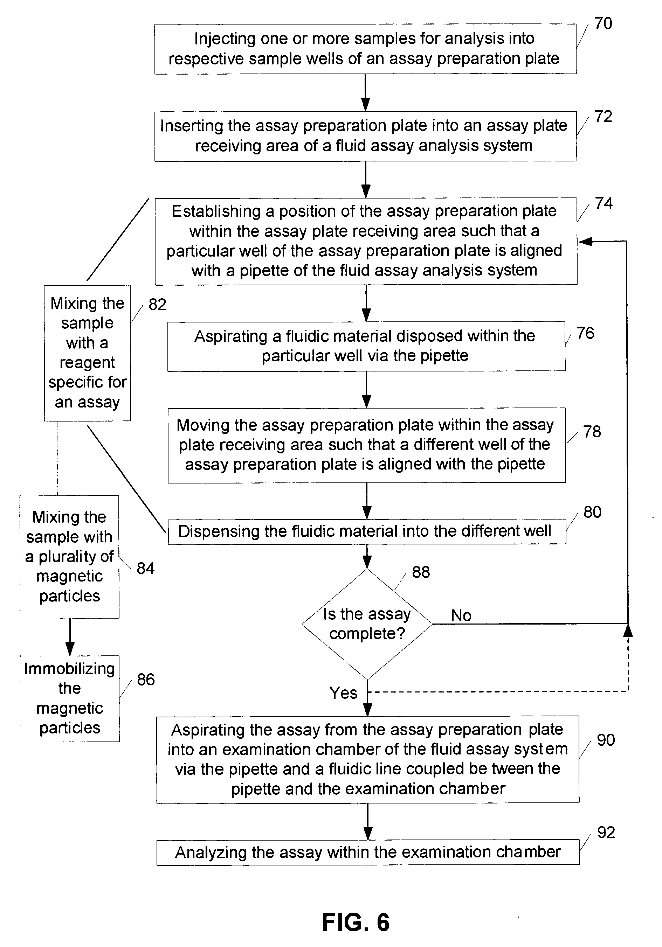

Turning to the drawings, exemplary embodiments of assay preparation plates, fluid assay systems, and methods for preparing and analyzing assays are shown. In particular, Fig. 1 illustrates exemplary fluid assay analysis system 10 configured to receive assay preparation plate 14. As set forth in more detail below, assay preparation plate 14 and/or fluid assay analysis system 10 may be configured to allow assays to be processed with magnetic particles within an assay preparation plate at the fluid assay analysis system through the automated use of pipette 12 and a magnet actuator. As a result, labor required to manually prepare an assay as well as error occurring from manual preparation may be reduced. Exemplary configurations of assay preparation plate 14 allowing assays to be processed at fluid assay analysis system 10 are shown in Figs. 2-4B and described in more detail below. An exemplary configuration of fluid assay analysis system 10 allowing assays to be processed at the system is shown in Figs. 5A and 5B and is described in more detail below. Fig. 6 illustrates a method for preparing and analyzing an assay using any of such configurations. It is noted that the figures are not necessarily drawn to scale. In particular, the scale of some elements in some of the figures may be greatly exaggerated to emphasize characteristics of the elements. In addition, it is further noted that the figures are not drawn to the same scale.

In general, fluid assay analysis system 10 may be configured to analyze a fluid assay. Such configurations include an examination chamber and a detection system for generating data representative of the presence, absence, and, in some embodiments, concentration of one or more analytes in an assay. In order to introduce an assay into fluid assay analysis system 10, the system may further include an assay plate receiving area and pipette 12 disposed above the assay plate receiving area for aspirating an assay from an assay plate. It is noted that Fig. 1 shows assay plate 14 only partially inserted into fluid assay analysis system 10 (i.e., the assay plate receiving area of fluid assay analysis system is generally beneath pipette 12). In order to aspirate an assay from assay plate 14 into fluid assay analysis system 10, the assay plate is inserted further such that a well of the assay plate containing an assay is disposed directly beneath pipette 12. Thereafter, pipette 12 moves downward to aspirate the assay and route it to an examination chamber of the fluid assay analysis system. In general, pipette 12 is coupled to the examination chamber via a fluidic line internal to fluid assay analysis system 10. In many instances, multiple assays are included in a single assay plate and, thus, fluid assay analysis system 10 may, in some embodiments, include a mechanism for moving an assay plate disposed within the assay plate receiving area such that different wells of the assay plate are aligned with the pipette at different times.

In some cases, fluid assay analysis system 10 may be an optical system and, thus, may include an illumination system configured to illuminate an examination chamber of the analysis system. In further embodiments, fluid assay analysis system 10 may be configured to optically analyze a particle-based assay. In such cases, fluid assay analysis system 10 may include a detection system configured to collect light emitted and/or scattered from assay particles and generate signals representative of a degree of light gathered. In addition, fluid assay analysis system 10 may include an examination system for analyzing the generated signals. Exemplary optical analysis systems having such components and which may be particularly applicable for the methods, systems, and devices described herein include flow cytometers and systems which immobilize particles for examination, such as static imaging systems. Both types of systems include a fluidic handling system for transporting a fluid assay and possibly other fluids to a particle examination chamber (and, thus, may be referred to as fluid assay systems). A multitude of flow cytometer configurations are known and may generally be applicable for the systems described herein. Exemplary static imaging optical analysis systems are described in the U.S. Patent Application 11/757,841 entitled "Systems and Methods for Performing Measurements of One or More Materials" by Roth et al. filed on June 4, 2007.

As mentioned above, the methods, systems, and devices described herein generally relate to configurations allowing assays to be processed with magnetic particles within an assay preparation plate at a fluid assay analysis system. More specifically, the methods, systems, and devices described herein relate to configurations which allow magnetic particles to be immobilized in a well of an assay preparation plate at a fluid assay analysis system for the purpose of preparing an assay. It is noted that the magnetic particles used to prepare an assay may or may not be included in the final assay product. In particular, magnetic particles may, in some cases, be discarded during the preparation of the assay. Alternatively, magnetic particles used for the preparation of an assay may be retained in the assay. Such specificity may generally depend on the specifications of the assay as well as the system used to analyze the assay. As noted above, fluid analysis assay system 10 may, in some embodiments, be configured to optically analyze particles included in an assay. Such particles, however, may or may not be magnetic particles. In particular, the specificity of whether particles in a final assay are magnetic may generally depend on the specifications of the assay as well as the system used to analyze the assay, regardless of whether magnetic particles are used to process a sample into an assay.

Regardless of whether a particle is magnetic or not, the term "particle" is used herein to generally refer to microspheres, polystyrene beads, quantum dots, nanodots, nanoparticles, nanoshells, beads, microbeads, latex particles, latex beads, fluorescent beads, fluorescent particles, colored particles, colored beads, tissue, cells, micro-organisms, organic matter, non-organic matter, or any other discrete substrates or substances known in the art. Any of such terms may be used interchangeably herein. Exemplary magnetic microspheres which may be used for the methods and systems described herein include xMAP® microspheres, which may be obtained commercially from Luminex Corporation of Austin, Texas.

It is noted that the processing or preparation of assays referred to herein may refer a wide scope of processing steps. In particular, assay processing or preparation may, in some embodiments, refer to converting a raw sample (e.g., blood or saliva) into a form that is compatible with a desired assay. As one skilled in the art is aware, different fluids may necessitate different processing steps and/or a different sequence of processing steps to achieve an assay and, thus, conversion of a raw sample may refer to a wide scope of processing steps. The processing steps may include any one or more of particle size filtering, centrifuging, analyte isolation, analyte amplification, washing of the sample, cell lysing, clotting factor neutralization, pH regulation, temperature cycling, reagent mixing, and assay reaction. Other processing steps may be considered as well. In other embodiments, processing or preparing an assay may refer to converting a partially-processed sample (i.e., a sample which one or more of the aforementioned processing steps has been already performed) into an assay. In any case, the sample may include any biological, chemical, or environmental fluid in which determination of the presence or absence of one or more analytes of interest is desired.

As noted above, assay preparation plate 14 may, in some embodiments, be configured to allow assays to be processed with magnetic particles within an assay plate receiving area of fluid assay analysis system 10. In particular, assay preparation plate 14 may, in some embodiments, may include an array of wells, a magnet, and an actuator configured to move the magnet proximate and remote relative to one or more select wells of the array of wells. Exemplary embodiments of assay preparation plates having such configurations are shown in Figs. 2-4B and described in more detail below. In such cases, fluid assay analysis system 10 may include an assay plate receiving area and a mechanism for moving an assay plate within the assay plate receiving area such that different wells of the assay plate are vertically aligned with the pipette at different times. In this manner, the pipette may be used to transfer fluidic material (i.e., reagents and/or samples) among different wells of the assay plate to prepare one or more assays.

It is noted that the components of a pipette, an assay plate receiving area, and a mechanism for moving an assay plate within the receiving area may be common in conventional systems. In particular, such a collection of components are generally used to aspirate multiple assays from a single assay plate into a fluid assay analysis system. The distinction set forth in the systems described herein is that such components are used for the preparation of an assay as well as to aspirate assays into a fluid assay system. In general, fluidic assay analysis system 10 may include a storage medium with program instructions which are executable by a processor to execute the movement of an assay plate (via the mechanism for moving an assay plate included in the fluid assay analysis system) and the pipette to accomplish the assay preparation. In some cases, the fluidic assay analysis system 10 may be retrofitted with the software to accommodate assay preparation at its assay plate receiving area. In this manner, the assay preparation plates described herein may be used with any existing fluid assay analysis systems having a pipette and an assay plate receiving area.

Turning to Figs. 2 and 3, exemplary embodiments of exterior configurations of assay preparation plates 20 and 30 are respectively shown. As shown in Fig. 2, assay preparation plate 20 includes circular sample wells 22, oblong reagent wells 24, and rectangular auxiliary wells 25. The shapes of the wells do not generally contribute to the preparation of an assay and, thus, may be altered from what is depicted in Fig. 2. Sample wells 22 may generally serve to receive sample fluids prior to the assay plate being placed in a fluid assay analysis system. Such sample fluids may include raw sample fluids or partially processed sample fluids. Reagent wells 24 may each include a reagent for processing the sample fluids received in sample wells 22 and, in some embodiments, may be dimensionally designed to store an amount of a reagent used for preparation of a single assay. Auxiliary wells 25 may generally serve to store or receive relatively large amounts of fluidic material, such as reagents common to all assays prepared in the plate (reagent bulk storage) and/or waste material resulting from the assay preparations.

The term "reagent" may generally be used herein to refer to a substance used to prepare an assay, including but not limited to magnetic particles. In some cases, some of the reagents may be lyophilized, particularly for field use where refrigeration is not available. In such cases, it may be advantageous for reagent wells 24 to have a relatively small volume. In particular, a more uniform and reliable re-suspension is possible using a smaller volume to re-suspend the lyophilized reagents. In some embodiments, the sample fluids may be used to re-suspend the reagents, which may advantageously keep consumables use down. In some embodiments, however, the reagents held in assay preparation plate 20 may not be lyophilized. Such a scenario may be particularly suitable for a laboratory environment where refrigerated storage is available.

As apparent to one skilled in the art, the number, size, and layout of wells 22, 24, and 25 may vary greatly and, thus, the depiction of the assay preparation plates described herein are not limited to the depiction of Fig. 2. As described in more detail below in reference to Figs. 4A and 4B, the general layout configuration of samples wells 22 and reagent wells 24 may, in some embodiments, be advantageous for the type of magnet assembly system discussed in reference to those figures. In particular, it may, in some cases, be advantageous for samples wells 22 and reagent wells 24 to be arranged in rows with alternating position of the different wells. However, various other magnet systems may be employed in the assay preparation plates described herein and, thus, the plates are not restricted to the layout configuration depicted in Fig. 2.

In addition to wells 22, 24, and/or 25, assay preparation plate 20 may include casing 26. Casing 26 generally provides a case to hold wells 22, 24, and 25 and is dimensionally configured to fit or mate into an assay plate receiving area of a fluid assay analysis system. In some cases, casing 26 further serves a sheath over other components of the assay preparation plate, such as those described in reference to Figs. 4A and 4B. In such cases, casing 26 may be generally designed for reuse (e.g., formed of a durable material) since the underlying components may be costly. In some cases, wells 22, 24, and/or 25 may be permanently fixed within casing 26 (i.e., wells 22, 24, and/or 25 may be made of the same contiguous material as casing 26 or the material comprising wells 22, 24, and/or 25 may be permanently fixed within casing 26). In other embodiments, however, wells 22, 24, and/or 25 may be disposed in removable inserts fastened within casing 26. In such cases, the removable inserts may, in some embodiments, be discarded after use and replacement inserts may be inserted into casing 26 for subsequent assay preparation. Alternatively, the removable inserts (as well as casing 26) may be cleaned and sanitized for reuse. In any case, wells 22, 24, and/or 25 may, in some embodiments, be encapsulated with frangible covers prior to assay preparation to avoid the wells from being contaminated with foreign substances and the reagents from spilling out of the assay plate. The frangible covers may generally be pierce-able by any device used to introduce or draw out fluids to and from the wells, such as pipette 12 of fluid assay analysis system 10.

As shown in Fig. 2, assay preparation plate 20 may further include probe sensor 28. Probe sensor 28 may generally be used to activate one or more magnet actuators disposed within assay preparation plate 20 such that magnetic particles within sample wells 22 and reagents wells 24 may be manipulated (i.e., immobilized and mobilized) for the preparation of an assay in the wells. In particular, assay preparation plate 28 may include one or more circuits coupling probe sensor 28 to the one or more magnet actuators, the one or more actuation circuits being configured to respectively activate the magnet actuator/s to move one or more magnets in proximity or remote to sample wells 22 and/or reagent wells 24 upon probe sensor 28 detecting a probe (e.g., pipette 12 of fluid assay analysis system 10). The circuit may be disposed in a printed circuit board assembly (PCBA) included in assay preparation plate 20 beneath casing 26, such as PCBA 52 shown in Fig. 4B. In general, probe sensor 28 may include any number of sensor technologies, such as but not limited to a capacitive proximity sensor, optical gate, physical completion of an electrical circuit, acoustic reflections, or magnetic field perturbation. Furthermore, although probe sensor 28 is illustrated as a slot in assay preparation plate 20, other configurations are possible. Alternatively, probe sensor 28 and the actuation circuit/s may be omitted from assay preparation plate 20 in some embodiments. In particular, a control line may alternatively be used to couple assay preparation plate 20 to fluid assay analysis system 10 such that the magnetic actuator may be directly activated via software included in the fluid assay analysis system (i.e., similar to the software used to control the movement of pipette 12 and a plate within its assay preparation plate receiving area).

The general operation of probe sensor 28 and the one or more actuation circuits to activate the one or more magnet actuators may generally include moving assay preparation plate 20 within an assay preparation plate receiving area of fluid assay analysis system 10 so that probe sensor 28 is in alignment with pipette 12. An initialization routine, such as lowering and raising pipette 12 twice rapidly, may be performed to ensure that the assay preparation plate 20 and pipette 12 are both in their proper positions. Once assay preparation plate 20 is in the correct position, pipette 12 is lowered as though it were aspirating fluid. Probe sensor 28 detects the proximity of the pipette and the position of a magnet actuator is changed via a circuit coupling the probe sensor 28 to the magnet actuator. The process of lowering pipette 12 proximate to the probe sensor 28 is generally repeated each time a magnet position needs to be changed. In some cases, assay preparation plate 20 may include a single actuation circuit, which is either configured to activate a single magnetic actuator or a plurality of magnetic actuators at the same time. In yet other cases, assay preparation plate 20 may include multiple actuation circuits for respectively actuating different magnet actuators disposed beneath casing 26. Such selectivity may be facilitated by incorporating multiple sensors within assay preparation plate 20 (i.e., in the vicinity of probe sensor 28 or in other locations of casing 26) that are respectively coupled to the multiple actuation circuits and software within fluid assay analysis system 10 that accurately positions pipette 12 relative to the different positions of the multiple sensors.

Although not shown in Fig. 2, assay preparation plate 20 may, in some embodiments, include indicators or controls included within or sticking out through casing 26. The controls may include configurations for scrolling through status messages and/or turning power to the plate on and off. The indicators may be used to alert a user of fluid assay analysis system 10 regarding the status of assay preparation (e.g., in-process, completed, and/or if an error occurred) and/or battery level (if applicable). The indicators may include any type of display known to those in the art, including but not limited to light-emitting diodes (LED), an acoustic transducer, or an alpha numeric display. In some cases, battery level and/or status notifications may be additionally or alternatively passed up through a control line coupling assay preparation plate 20 to fluid assay analysis system 10. As such, assay preparation plate 20 may not include indicators and/or controls in some embodiments.

An alternative configuration of an assay preparation plate is shown in Fig. 3. In particular, Fig. 3 illustrates assay preparation plate 30 including sample wells 32, reagent wells 34, and waste well 35 disposed within casing 36. In general, the characteristics of casing 36 and wells 32, 34, and 35 may be similar to those described for casing 26 and wells 22, 24, and 25 of assay preparation plate 20 in Fig. 2. The descriptions are not reiterated for the sake of brevity and, thus, are referenced herein as if set forth in full. As discussed with respect to assay preparation plate 20 depicted in Fig. 2, the shape, size, number, and layout of wells 32, 34, and 35 may vary widely and, thus, the assay preparation plates discussed herein should not be limited to the illustration of Fig. 3. Although not necessarily so limited, assay preparation plate 30 is generally configured to process assays sequentially in each row of sample wells 32. In particular, each of sample wells 1-12 may be used to process a sample with a different reagent and each row of sample wells A-D is used to process a different sample, resulting in a different assay for each of rows A-D. Alternatively, assays may be processed in a subset of the sample wells in a row or in a column of wells 32. In yet other embodiments, assays may be processed in a single well within assay preparation plate 30. Assay preparation plate 20 depicted in Fig. 2 may be used in similar manners and, in some embodiments (although not necessarily so limited), may be particularly applicable for processing an assay in a single well.

In addition to casing 36 and wells 32, 34, and 35, assay preparation plate 30 may include other components, such as but not limited to the components described above and below for assay preparation plate 20. In particular, assay preparation plate 30 may include components underlying casing 36, such as but not limited magnet/s, magnet actuator/s, a battery, a PCBA, and a control switch. In addition, assay preparation plate 30 may include indicators, controls, probe sensor/s and accompany actuation circuit/s. The descriptions are not reiterated for the sake of brevity and, thus, are referenced herein as if set forth in full.

As noted above, exemplary configurations of the interior components of assay preparation plate 20 are illustrated in Figs. 4A and 4B. In particular, Figs. 4A and 4B depict an exemplary layout of three magnet assemblies each including magnets 42 and common bar 44. Fig. 4A illustrates a perspective view of assay preparation plate 20 with casing 26 removed and Fig. 4B illustrates a top view of assay preparation plate 20 with casing 26 removed. Magnets 42 generally extend beneath or juxtapose to a neighboring row of wells such that magnetic particles therein may be immobilized. The three magnet assemblies are respectively coupled to magnet actuators 45-47, which are configured to move magnets 42 of each assembly proximate and remote relative to select sample wells. In particular, as shown in Fig. 4B, magnet actuators 45 and 47 are retracted such that magnets 42 of the magnet assemblies attached thereto are aligned with select sample wells and, thus, the magnets are in position to immobilize magnetic particles in the select sample wells. On the contrary, magnet actuator 46 is extended such that magnets 42 of the magnet assembly attached thereto are offset from select sample wells and, more specifically, aligned with neighboring reagent wells. In such cases, magnetic particles in the select sample wells are not immobilized. It is noted that the positions of magnets 42 depicted in Fig. 4B to be aligned with sample wells or reagents wells relative to whether magnet actuators 45-47 are extended or retracted may be reversed. In either case, as shown in Fig. 4B, magnets 42 may be uniformly arranged relative to the spacings of sample wells 22. In this manner, magnets 42 of a single magnet assembly may be moved in unison proximate and remote to the sample wells.

Although assay preparation plate 20 is shown in Fig. 4B to include three distinct magnet assemblies and three distinct magnet actuators, the assay preparation plates described herein are not necessarily so limited. In particular, the assay preparation plates described herein may include fewer or more magnet assemblies and/or magnet actuators. For example, assay preparation plate 20 may be modified to include a single magnet actuator coupled to each of the three magnet assemblies such that the magnets of the magnet assemblies may be moved collectively. Alternatively, the magnet assemblies of assay preparation plate 20 may be modified to be a single magnet assembly. In particular, magnets 42 may include rods extending through the three common bars shown. In such cases, a single magnet actuator may be used to collectively move magnets 42 proximate and remote to sample wells 22.

In yet other embodiments, assay preparation plate 20 may not include magnet assemblies. Rather, assay preparation plate 20 may include one or more individual magnets with one or more corresponding magnet actuators. Furthermore, the assay preparation plates described herein are not necessarily limited to having magnet actuators arranged to horizontally displace magnets relative to wells of the assay preparation plate as depicted in Fig. 4B. In particular, the assay preparation plates described herein may include configurations of magnet actuators which move magnets in a vertical direction. In such cases, when the magnet actuators are retracted, the magnets may be disposed a sufficient distance below the sample wells of the assay preparation plate such that magnetic particles disposed therein are not immobilized. Conversely, when the magnet actuators are extended, the magnets may be proximate to the sample wells such that magnetic particles disposed therein are mobilized.

In general, the magnet actuators included in the assay preparation plates described herein may include any type of actuator, including but not limited to ones driven by mechanical means, electrical means, pneumatic means, or magnetic means.

In any case, in addition to magnets and magnet actuators, assay preparation plate 20 includes PCBA 50, battery 52, control switch 54, and indicators 56. Onboard battery 52 can be supplemented or substituted by a power line coupled between assay preparation plate 20 and fluid assay analysis system 10. PCBA 50 includes but is not limited to a circuit for controlling the magnet actuators 35-37 and a circuit for charging battery 50 (when applicable). Battery charging can be performed through direct conduction through electrodes, a charging cable, or an inductive coil. Control switch 42 may generally be used to turn power to the plate on and off. Indicators 56 are shown to specifically denote light emitting diodes, but other types of indicators may additionally or alternatively be employed as described above in reference to Fig. 2.

As noted above, fluid assay analysis system 10 may, in some embodiments, be configured to allow assays to be processed with magnetic particles within an assay preparation plate area of the system (i.e., rather than an assay preparation plate being configured to do so). Partial schematic drawings of an exemplary fluid assay analysis system having such a configuration are illustrated in Figs. 5A and 5B. In particular, Fig. 5A illustrates a partial schematic drawing of fluid assay preparation and analysis system 60 having magnet actuator 66 disposed below and in approximate alignment with pipette 62, having magnet 68 retracted within magnet actuator 66. Fig. 5B illustrates a partial schematic view of fluid assay preparation and analysis system 60 in which magnet actuator 66 has moved magnet 68 in the vicinity of assay plate receiving area 64 interposed between pipette 62 and magnet actuator 68. In this manner, magnet actuator 66 is configured to move magnet 68 to and from a position proximate assay plate receiving area 64. Consequently, magnetic particles disposed within a well of an assay preparation plate which is aligned with pipette 62 and magnet 68 may be immobilized as well as released from immobilization. In particular, with the placement and orientation of magnet actuator 66 and magnet 68, magnetic particles may be immobilized at the bottom of a well. As a consequence, excess fluid can be aspirated from the well.

As set forth above for fluid assay analysis system 10, fluid assay analysis system 60 may further include a mechanism for moving an assay plate disposed within assay plate receiving area 64 such that different wells of the assay plate are aligned with pipette 12 at different times. Such a configuration may allow multiple reagents to be mixed with a sample for preparation of an assay. In addition, the mechanism for moving an assay plate within assay plate receiving area 64 may allow multiple assays to be prepared in a single assay plate. In general, fluidic assay analysis system 60 may include a storage medium with program instructions which are executable by a processor to execute the movement of an assay plate within assay plate receiving area 64 (via the mechanism for moving an assay plate arranged in the receiving area) as well as movement of pipette 12 to accomplish the assay preparation. In addition, the storage medium may include program instructions for selectively activating magnet actuator 66.

In addition to having the ability to prepare one or more assays through the incorporation of magnet actuator 66 and magnet 68, fluid assay analysis system 60 is also configured to analyze fluid assays. In this manner, fluid assay analysis system is configured to both prepare and analyze a fluid assay and, thus, may be referred to as a fluid assay preparation and analysis system. As such, fluid assay analysis system 60 may further include (as discussed with respect to fluid assay analysis system 10 in Fig. 1) an examination chamber coupled to pipette 12 via a fluidic line and a detection system for generating data representative of the presence, absence, and, in some embodiments, concentration of one or more analytes in an assay. In some cases, fluid assay analysis system 60 may be an optical system and, thus, may include an illumination system configured to illuminate the examination chamber. In further embodiments, fluid assay analysis system 60 may be configured to optically analyze a particle-based assay. In such cases, fluid assay analysis system 60 may include a detection system configured to collect light emitted and/or scattered from assay particles and generate signals representative of a degree of light gathered. In addition, fluid assay analysis system 60 may include an examination system for analyzing the generated signals. Exemplary optical analysis systems having such components and which may be particularly applicable for fluid assay analysis system 60 include flow cytometers and systems which immobilize particles for examination, such as static imaging systems. Both types of systems include a fluidic handling system for transporting a fluid assay and possibly other fluids to a particle examination chamber (and, thus, may be referred to as fluid assay systems).

As discussed with regard to magnet actuators 45-47 in Fig. 4B, magnet actuator 66 may include any type of actuator, including but not limited to ones driven by mechanical means, electrical means, pneumatic means, or magnetic means. However, magnet actuator 66 should not be construed to necessarily be limited to such an actuator. Furthermore, magnet actuator 66 is not limited to an orientation which facilitates vertical movement of magnet 66 in proximity and remote to assay receiving plate area 64. In particular, magnet actuator 66 may alternatively be employed to cause horizontal movement of magnet to immobilize magnetic particles within a well of an assay preparation plate.

A flowchart of a method for preparing and analyzing an assay is outlined in Fig. 6. As shown in block 70 of Fig. 6, the method includes injecting one or more samples for analysis into respective sample wells of an assay preparation plate. The one or more samples may include any biological, chemical, or environmental fluid in which determination of the presence or absence of one or more analytes of interest is desired. The process of injecting the one or more samples may be performed manually or through automation, but in either case is generally conducted prior to inserting the assay preparation plate into an assay plate receiving area of a fluid assay analysis system, a process of which is shown in block 72. After the assay preparation plate is placed into the assay plate receiving area, the method continues to block 74 at which a position of the assay preparation plate within the assay plate receiving area is established such that a particular well of the assay preparation plate is aligned with a pipette of the fluid assay analysis system. In some cases, the particular well may a reagent well. In other embodiments, however, the particular well may be one of the sample wells injected with the one or more samples, particularly in embodiments in which a sample well includes a reagent (e.g., magnetic particles or dilution agent) prior to the injection of a sample therein.

In either case, the method includes aspirating a fluidic material disposed within the particular well via the pipette and moving the assay preparation plate within the assay plate receiving area such that a different well of the assay preparation plate is aligned with the pipette as shown respectively in blocks 76 and 78. Thereafter, the method continues to block 80 in which the fluidic material is dispensed into a different well. The different well may be the sample well (i.e., the well having the originally injected sample) or may be a reagent well or a different sample well. In any case, the processes delineated in blocks 74, 76, 78, and 80 include mixing the sample with a reagent specific for an assay as denoted in block 82. As noted by the dotted line extension from block 82, the reagent may include a plurality of magnetic particles and, thus, the processes delineated in blocks 74, 76, 78, and 80 may include mixing the sample with magnetic particles shown in block 84. In such cases, as noted by block 86, the method may include immobilizing the magnetic particles, particularly at some point when the processes delineated in blocks 74, 76, 78, and 80 are performed. In some cases, as discussed above in reference to Fig. 2, the immobilization process may include moving the assay preparation plate within the assay plate receiving area such that the pipette is aligned with a probe sensor of the assay preparation plate and lowering the pipette down to the probe sensor. Upon detecting the pipette with the probe sensor, the assay preparation plate may be moved within the assay plate receiving area such that the pipette is aligned with a well of the assay preparation plate comprising the magnetic particles and a magnet actuator may actuate a magnet in proximity to the well comprising the magnetic particles.

At block 88, a determination is made as to whether the assay is complete. If the assay is not complete the method returns to block 74 and repeats the processes delineated in blocks 74, 76, 78, and 80 until preparation of the assay is complete. It is noted that each pass through the processes delineated in blocks 74, 76, 78, and 80 need not necessarily include immobilizing magnetic particles or even mixing the sample with magnetic particles. In particular, the processing or preparation of an assay may refer a wide scope of processing steps and associated reagents. Other reagents which may additionally or alternatively be mixed into the sample may include those used for centrifuging, analyte isolation, analyte amplification, washing of the sample, cell lysing, clotting factor neutralization, pH regulation, temperature cycling, reagent mixing, and assay reaction. Reagents for other processing steps may be considered as well. Furthermore, it is noted that the processes delineated in blocks 74, 76, 78, and 80 may include preparing an assay in a single well, such as the sample well the sample was originally injected into, or may include preparing an assay using a plurality of wells and, in some embodiments, a series of sample wells aligned in an assay preparation plate.

Upon determining an assay is complete at block 88, the method may optionally return to block 74 as denoted by the dotted arrow line to prepare another assay with one of the other samples that was injected into the assay preparation plate at block 70. In this manner, the method may include serially preparing respective assays for each of samples injected into the assay preparation plate. In other embodiments, however, the method may include preparing respective assays for several samples injected into the assay preparation plate in parallel. Such an embodiment may be more efficient if the same assay preparation procedure is being conducted for several assays. In particular, the pipette of the fluid assay analysis system may be used to aspirate a relatively large quantity of reagent and distribute it to each of the samples.

In any case, the method further includes analyzing the one or more fluid assays and, thus, includes aspirating a prepared assay from the assay preparation plate into an examination chamber of the fluid assay system via the pipette and a fluidic line coupled between the pipette and the examination chamber and analyzing the prepared assay within the examination chamber as denoted in blocks 90 and 92. Such a sequence of steps may be repeated for each assay prepared.

It will be appreciated to those skilled in the art having the benefit of this disclosure that this invention is believed to provide assay preparation plates, fluid assay systems, and methods for preparing and analyzing assays which allow assays to be processed within an assay preparation plate by components of a fluid assay analysis system. For example, any type of magnet actuators may be used in the devices, systems, and methods described herein to move a magnet proximate and remote from a well of an assay preparation plate and, thus, the devices, systems, and methods described herein should not be limited to the depictions of magnet actuators in the figures. Accordingly, this description is to be construed as illustrative only and is for the purpose of teaching those skilled in the art the general manner of carrying out the invention. It is to be understood that the forms of the invention shown and described herein are to be taken as the presently preferred embodiments. Elements and materials may be substituted for those illustrated and described herein, parts and processes may be reversed, and certain features of the invention may be utilized independently, all as would be apparent to one skilled in the art after having the benefit of this description of the invention.

## Claims

1. An assay preparation plate, comprising:
- an array of wells;
- a magnet; and
- an actuator configured to move the magnet proximate and remote relative to one or more select wells of the array of wells.

2. The assay preparation plate of claim 1, wherein the magnet is one of a plurality of magnets comprising the assay preparation plate.

3. The assay preparation plate of claim 2, wherein at least some of the plurality of magnets are uniformly positioned relative to the select wells or wherein the actuator is one of a plurality of actuators respectively configured to move the plurality of magnets proximate and remote relative to the select wells or wherein the plurality of magnets comprise one or more assemblies of magnets.

4. The assay preparation plate of the previous claim, wherein in the case that the plurality of magnets comprise one or more assemblies of magnets the actuator is configured to collectively move the one or more assemblies such that the plurality of magnets are proximate or remote relative to the select wells.

5. The assay preparation plate of claim 1, wherein the magnet is disposed below the array of wells or wherein the magnet is juxtapose to one or more wells of the array of wells.

6. The assay preparation plate of claim 1, further comprising:
- a probe sensor; and
- a circuit coupling the probe sensor to the actuator, wherein the circuit is configured to activate the actuator when a probe is detected by the probe sensor.

7. The assay preparation plate of claim 1, wherein some of the wells of the array of wells comprise reagents for preparing an assay.

8. The assay preparation plate of the previous claim, wherein at least one of the reagents is a set of magnetic particles.

9. The assay preparation plate of claim 1, wherein the array of wells is disposed in removable inserts fastened within the assay preparation plate.

10. A method for preparing and analyzing an assay, comprising:
- providing an assay preparation plate according to claim 1,
- injecting a sample for analysis into a sample well of the assay preparation plate;
- inserting the assay preparation plate into an assay plate receiving area of a fluid assay analysis system;
- establishing a position of the assay preparation plate within the assay plate receiving area such that a particular well of the assay preparation plate is aligned with a pipette of the fluid assay analysis system;
- aspirating a fluidic material disposed within the particular well via the pipette;
- moving the assay preparation plate within the assay plate receiving area such that a different well of the assay preparation plate is aligned with the pipette;
- dispensing the fluidic material into the different well, wherein the steps of establishing, aspirating, moving, and dispensing comprise mixing the sample with a reagent specific for an assay;
- repeating the steps of establishing, aspirating, moving, and dispensing to mix the sample with one or more additional reagents until preparation of the assay is complete, wherein at least one series of the steps of establishing, aspirating, moving, and
- dispensing comprises mixing the sample with a plurality of magnetic particles;
- immobilizing the plurality of magnetic particles in a well of the assay preparation plate subsequent to mixing the sample with the plurality of magnetic particles;
- aspirating the assay from the assay preparation plate into an examination chamber of the fluid assay system via the pipette and a fluidic line coupled between the pipette and the examination chamber; and
- analyzing the assay within the examination chamber.

11. The method of claim 10, wherein the steps of establishing, aspirating, moving, and dispensing comprise preparing the assay in the sample well or wherein the steps of establishing, aspirating, moving, and dispensing comprise preparing the assay in a series of wells within the assay preparation plate or wherein the step of immobilizing the plurality of magnetic particles comprises:.
- moving the assay preparation plate within the assay plate receiving area such that the pipette is aligned with a probe sensor of the assay preparation plate;
- lowering the pipette down to the probe sensor; and
- upon detecting the pipette with the probe sensor:
moving the assay preparation plate within the assay plate receiving area such that the pipette is aligned with the well of the assay preparation plate comprising the magnetic particles; and
actuating a magnet in proximity to the well comprising the magnetic particles.

12. The method of claim 10, further comprising respectively injecting one or more additional samples for analysis into other sample wells of the assay preparation plate prior to the step of inserting the assay preparation plate into an assay plate receiving area.

13. The method of claim 12, wherein the steps of establishing, aspirating, moving, and dispensing further comprise preparing respective assays for each of one or more additional samples in parallel with the preparation of the assay or wherein the method further comprises reiterating the steps of establishing, aspirating, moving, and dispensing to serially prepare respective assays for each of one or more additional samples.

14. A fluid assay preparation and analysis system, comprising:
- an assay preparation plate according to claim 1,
- an assay plate receiving area;
- a pipette disposed above the assay plate receiving area;
- a magnet disposed below the assay plate receiving area in approximate alignment with the pipette;
- an actuator configured to move the magnet to and from a position proximate the assay plate receiving area;
- a mechanism for moving an assay plate disposed within the assay plate receiving area such that different wells of the assay plate are aligned with the pipette at different times;
- an examination chamber coupled to the pipette via a fluidic line;
- an illumination system configured to illuminate the examination chamber;
- a detection system configured to collect light emitted and/or scattered from assay particles introduced into the examination chamber via the pipette and the fluidic line, wherein the detection system is further configured to generate signals representative of a degree of light gathered; and
- an examination system for analyzing the generated signals.

15. The fluid assay preparation and analysis system of claim 14, further comprising a storage medium with program instructions which are executable by a processor for selectively activating the actuator or wherein the fluid assay preparation and analysis system comprises a flow cytometer or a static imaging optical system.

## Patentansprüche

1. Proben-Zubereitungsplatte, umfassend:
- eine Anordnung von Kavitäten ("Wells");
- einen Magneten; und
- ein Aktor, der konfiguriert ist, um den Magneten in die Nähe einer oder mehrerer ausgewählter Kavitäten der Anordnung von Kavitäten zu bewegen oder davon zu entfernen.

2. Proben-Zubereitungsplatte gemäß Anspruch 1, wobei der Magnet einer aus einer Mehrzahl von Magneten ist, welche die Assay-Zubereitungsplatte aufweisen.

3. Proben-Zubereitungsplatte gemäß Anspruch 2, wobei wenigstens einige der Mehrzahl von Magneten im Verhältnis zu den ausgewählten Kavitäten gleichförmig positioniert sind oder wobei der Aktor einer aus einer Mehrzahl von Aktoren ist, die jeweils so konfiguriert sind, dass sie die Mehrzahl von Magneten in die Nähe der ausgewählten Kavitäten bewegen oder davon entfernen, oder wobei die Mehrzahl von Magneten eine oder mehrere Gruppen von Magneten umfassen.

4. Proben-Zubereitungsplatte gemäß dem vorangehenden Anspruch, wobei für den Fall, dass die Mehrzahl von Magneten eine oder mehrere Gruppen von Magneten umfassen, der Aktor so konfiguriert ist, dass er die eine oder mehreren Gruppen kollektiv bewegt, so dass die Mehrzahl von Magneten in die Nähe der ausgewählten Kavitäten bewegt oder davon entfernt werden.

5. Proben-Zubereitungsplatte gemäß Anspruch 1, wobei der Magnet unterhalb der Anordnung von Kavitäten angeordnet ist oder wobei der Magnet einer oder mehreren Kavitäten der Anordnung von Kavitäten beigeordnet ist.

6. Proben-Zubereitungsplatte gemäß Anspruch 1, ferner umfassend:
- einen Sensor zur Erfassung einer Probe; und
- einen Schaltkreis, der den Sensor mit dem Aktor koppelt, wobei der Schaltkreis dazu konfiguriert ist, den Aktor zu aktivieren, wenn der Sensor eine Probe erfasst.

7. Proben-Zubereitungsplatte gemäß Anspruch 1, wobei einige der Kavitäten der Anordnung von Kavitäten Reagenzien zur Zubereitung einer Probe umfassen.

8. Proben-Zubereitungsplatte gemäß dem vorhergehenden Anspruch, wobei wenigstens eine der Reagenzien ein Satz magnetischer Partikel ist.

9. Proben-Zubereitungsplatte gemäß Anspruch 1, wobei die Anordnung von Kavitäten in herausnehmbaren Einsätzen angeordnet ist, die in der Assay-Zubereitungsplatte befestigt sind.

10. Verfahren zur Herstellung und Analyse einer Probe, umfassend:
- Bereitstellen einer Proben-Zubereitungsplatte gemäß Anspruch 1;
- Einspritzen einer Probe zur Analyse in eine Proben-Kavität der Proben-Zubereitungsplatte;
- Einsetzen der Proben-Zubereitungsplatte in einen Probeplatten-Aufnahmebereich eines Fluid-Proben-Analysesystems;
- Einrichten einer Position der Proben-Zubereitungsplatte im Probeplatten-Aufnahmebereich, so dass eine bestimmte Kavität der Proben-Zubereitungsplatte an einer Pipette des Fluid-Proben-Analysesystems ausgerichtet ist;
- Ansaugen eines Fluidmaterials welches in die bestimmte Kavität eingelegt wurde über die Pipette;
- Bewegen der Proben-Zubereitungsplatte innerhalb des Probeplatten-Aufnahmebereichs, so dass eine andere Kavität der Proben-Zubereitungsplatte an der Pipette ausgerichtet wird;
- Abgeben des Fluidmaterials in die andere Kavität, wobei die Schritte des Einrichtens, Ansaugens, Bewegens und Abgebens das Mischen der Probe mit einer probenspezifischen Reagens umfassen;
- Wiederholen der Schritte des Einrichtens, Ansaugens, Bewegens und Abgebens, um die Probe mit einem oder mehreren zusätzlichen Reagenzien zu mischen, bis die Zubereitung der Probe abgeschlossen ist, wobei mindestens eine Reihe der Schritte des Einrichtens, Ansaugens, Bewegens und Abgebens das Mischen der Probe mit einer Mehrzahl magnetischer Partikel umfasst;
- Immobilisieren der Mehrzahl magnetischer Partikel in einer Kavität der Proben-Zubereitungsplatte nach dem die Probe mit der Mehrzahl der magnetischer Partikel gemischt wurde;
- Ansaugen der Probe aus der Proben-Zubereitungsplatte in eine Prüfkammer des Fluid-Proben-Systems über die Pipette und eine Fluidleitung, die zwischen der Pipette und der Prüfkammer gekoppelt ist; und
- Analysieren der Probe in der Prüfkammer.

11. Verfahren gemäß Anspruch 10, wobei die Schritte des Einrichtens, Ansaugens, Bewegens und Abgebens das Zubereiten der Probe in der Proben-Kavität umfassen oder wobei die Schritte des Einrichtens, Ansaugens, Bewegens und Abgebens das Zubereiten der Probe in einer Reihe von Kavitäten in der Proben-Zubereitungsplatte umfassen oder wobei der Schritt der Immobilisierung der Mehrzahl magnetischer Partikel umfasst:
- Bewegen der Proben-Zubereitungsplatte innerhalb des Probeplatten-Aufnahmebereichs, so dass die Pipette an einem Sensor zur Erfassung einer Probe der Proben-Zubereitungsplatte ausgerichtet ist;
- Absenken der Pipette auf den Sensor; und
- nach der Erfassung der Pipette mit dem Sensor:
Bewegen der Proben-Zubereitungsplatte innerhalb des Assayplatten-Aufnahmebereichs, so dass die Pipette an der Kavität der Proben-Zubereitungsplatte, welche die magnetischen Partikel enthält, ausgerichtet ist; und Betätigen eines Magnets in der Nähe der Kavität, welche die magnetischen Partikel enthält.

12. Verfahren gemäß Anspruch 10, ferner umfassend das jeweilige Einspritzen einer oder mehrerer zusätzlicher Proben zur Analyse in andere Proben-Kavitäten der Proben-Zubereitungsplatte vor dem Schritt des Einfügens der Proben-Zubereitungsplatte in einen Probeplatten-Aufnahmebereich.

13. Verfahren gemäß Anspruch 12, wobei die Schritte des Einrichtens, Ansaugens, Bewegens und Abgebens ferner das Zubereiten entsprechender Proben für jede von einer oder mehreren zusätzlichen Proben parallel mit der Zubereitung der Probe umfassen oder wobei das Verfahren ferner die Wiederholung der Schritte des Einrichtens, Ansaugens, Bewegens und Abgebens zur seriellen Zubereitung entsprechender Proben für jede von einer oder mehreren zusätzlichen Proben umfasst.

14. Fluid-Proben-Zubereitungs- und Analysesystem, umfassend:
- eine Proben-Zubereitungsplatte gemäß Anspruch 1,
- einen Probenplatten-Aufnahmebereich;
- eine Pipette, die über dem Probenplatten-Aufnahmebereich angeordnet ist;
- einen Magneten, der unterhalb des Probenplatten-Aufnahmebereichs in annähernder Ausrichtung an der Pipette angeordnet ist;
- ein Aktor, der konfiguriert ist, um den Magneten in die Nähe des Probeplatten-Aufnahmebereichs zu bewegen und davon zu entfernen;
- einen Mechanismus zum Bewegen einer innerhalb des Probeplatten-Aufnahmebereichs angeordneten Probeplatte, so dass unterschiedliche Kavitäten der Probeplatte zu unterschiedlichen Zeiten an der Pipette ausgerichtet sind;
- eine Prüfkammer, die über eine Fluidleitung mit der Pipette gekoppelt ist;
- ein Beleuchtungssystem, das dazu konfiguriert ist, die Prüfkammer zu beleuchten;
- ein Detektionssystem, das dazu konfiguriert ist, Licht, das von den Probe-Partikeln, die über die Pipette und die Fluidleitung in die Prüfkammer eingeführt wurden, abgegeben und/oder gestreut wurde, zu sammeln, wobei das Detektionssystem ferner dazu konfiguriert ist, Signale abzugeben, die für einen Grad des erfassten Lichtes repräsentativ sind; und
- ein Prüfsystem zur Analyse der abgegebenen Signale.

15. Fluid-Proben-Zubereitungs- und Analysesystem gemäß Anspruch 14, ferner ein Speichermedium mit Programmanweisungen umfassend, die durch einen Prozessor zur selektiven Aktivierung des Stellgliedes ausführbar sind oder wobei das Fluidproben-Zubereitungs- und Analysesystem ein Durchflusszytometer oder ein statisches bildgebendes optisches System umfasst.

## Revendications

1. Une plaque de préparation d'essai, comprenant :
- un réseau de puits ;
- un aimant; et
- un actionneur configuré pour déplacer l'aimant à proximité et à distance par rapport à un ou plusieurs puits sélectionnés du réseau de puits.

2. La plaque de préparation d'essai de la revendication 1, où l'aimant fait partie d'une pluralité d'aimants comprenant la plaque de préparation d'essai.

3. La plaque de préparation d'essai de la revendication 2, où au moins certains aimants de la pluralité d'aimants sont uniformément positionnés par rapport aux puits sélectionnés ou bien où l'actionneur fait partie de la pluralité d'actionneurs configurés respectivement pour déplacer la pluralité d'aimants à proximité et à distance par rapport aux puits sélectionnés ou bien où la pluralité d'aimants comprend une ou plusieurs assemblées d'aimants.

4. La plaque de préparation d'essai de la revendication précédente, où dans le cas où la pluralité d'aimants comprend une ou plusieurs assemblées d'aimants, l'actionneur est configuré pour déplacer de façon collective un ou plusieurs assemblages de sorte que la pluralité d'aimants soit à proximité ou à distance par rapport aux puits sélectionnés.

5. La plaque de préparation d'essai de la revendication 1, où l'aimant est disposé sous le réseau de puits ou bien où l'aimant est juxtaposé à un ou plusieurs puits du réseau de puits.

6. La plaque de préparation d'essai de la revendication 1, comprenant de plus :
- un capteur à sonde ; et
- un circuit raccordant le capteur de sonde à l'actionneur, où le circuit est configuré pour activer l'actionneur lorsqu'une échantillon est détectée par le capteur de sonde.

7. La plaque de préparation d'essai de la revendication 1, où certains des puits du réseau de puits comprennent des réactifs pour préparer un essai.

8. La plaque de préparation d'essai de la revendication précédente, où au moins un des réactifs est un ensemble de particules magnétiques.

9. La plaque de préparation d'essai de la revendication 1, où le réseau de puits est disposé dans des inserts amovibles attachés dans la plaque de préparation d'essai.

10. Une méthode pour préparer et analyser un essai, comprenant :
- la fourniture d'une plaque de préparation d'essai selon la revendication 1,
- l'injection d'un échantillon pour analyse dans un puits d'échantillon de la plaque de préparation d'essai ;
- l'insertion de la plaque de préparation d'essai dans une zone de réception de la plaque d'essai d'un système d'analyse d'essai de fluide ;
- l'établissement d'une position de la plaque de préparation d'essai dans la zone de réception de la plaque d'essai de sorte qu'un puits particulier de la plaque de préparation d'essai soit aligné avec une pipette du système d'analyse d'essai du fluide ;
- l'aspiration d'un matériau liquide disposé dans le puits particulier via la pipette ;
- le déplacement de la plaque de préparation d'essai dans la zone de réception de la plaque d'essai de sorte qu'un puits différent de la plaque de préparation d'essai soit aligné avec la pipette ;
- la distribution du matériau liquide dans le puits différent, où les étapes d'établissement, d'aspiration, de déplacement et de distribution comprennent le mélange de l'échantillon avec un réactif spécifique pour un essai ;
- la répétition des étapes d'établissement, d'aspiration, de déplacement et de distribution pour mélanger l'échantillon avec un ou plusieurs réactifs jusqu'à ce que la préparation de l'essai soit complète, où au moins une série des étapes d'établissement, d'aspiration, de déplacement et
- la distribution comprend un mélange de l'échantillon avec une pluralité de particules magnétiques ;
- l'immobilisation de la pluralité des particules magnétiques dans un puits de la plaque de préparation d'essai postérieure au mélange de l'échantillon avec la pluralité de particules magnétiques ;
- l'aspiration de l'essai à partir de la plaque de préparation d'essai dans une chambre d'examen du système d'essai de fluide via la pipette et une ligne de liquide raccordée entre la pipette et la chambre d'examen ; et
- l'analyse de l'essai à l'intérieur de la chambre d'examen.

11. La méthode de la revendication 10, où les étapes d'établissement, d'aspiration, de déplacement et de distribution comprennent la préparation de l'essai dans le puits d'échantillons ou bien où les étapes d'établissement, d'aspiration, de déplacement et de distribution comprennent la préparation de l'essai dans une série de puits à l'intérieur de la plaque de préparation d'essai ou bien où l'étape d'immobilisation de la pluralité de particules magnétiques comprend :
- le déplacement de la plaque de préparation d'essai à l'intérieur de la zone de réception de la plaque d'essai de sorte que la pipette soit alignée avec un capteur à sonde de la plaque de préparation d'essai ;
- le rabaissement de la pipette en bas du capteur à sonde ; et
- lors de la détection de la pipette avec le capteur à sonde :
le déplacement de la plaque de préparation d'essai dans la zone de réception de la plaque d'essai de sorte que la pipette soit alignée avec le puits de la plaque de préparation d'essai comprenant les particules magnétiques ; et
l'action d'un aimant à proximité du puits comprenant les particules magnétiques.

12. La méthode de la revendication 10, comprenant de plus respectivement l'injection d'un ou de plusieurs échantillons supplémentaires pour une analyse dans d'autres puits d'échantillon de la plaque de préparation d'essai avant l'étape d'insertion de la plaque de préparation d'essai dans une zone de réception de la plaque d'essai.

13. La méthode de la revendication 12, où les étapes d'établissement, d'aspiration, de déplacement et de distribution comprennent de plus la préparation d'essais respectifs pour chacun d'un ou de plusieurs échantillons supplémentaires en parallèle avec la préparation de l'essai ou bien où la méthode comprend de plus la réitération des étapes d'établissement, d'aspiration, de déplacement et de distribution pour préparer en série des essais respectifs pour chacun d'un ou de plusieurs échantillons supplémentaires.

14. Un système d'analyse et de préparation d'essai de liquide, comprenant :
- une plaque de préparation d'essai selon la revendication 1,
- une zone de réception de la plaque d'essai ;
- une pipette disposée au-dessus de la zone de réception de la plaque d'essai ;
- un aimant disposé en-dessous de la zone de réception de la plaque d'essai dans un alignement approximatif avec la pipette ;
- un actionneur configuré pour déplacer l'aimant depuis et vers une position proche de la zone de réception de la plaque d'essai ;
- un mécanisme pour déplacer une plaque d'essai disposé dans la zone de réception de la plaque d'essai de sorte que différents puits de la plaque d'essai soient alignés avec la pipette à différents moments ;
- une chambre d'examen raccordée à la pipette via une ligne de liquide ;
- un système d'éclairage configuré pour éclairer la chambre d'examen ;
- un système de détection configuré pour récupérer la lumière émise et/ou éparpillée à partir des particules d'essai introduites dans la chambre d'examen via la pipette et la ligne de liquide, où le système de détection est de plus configuré pour générer des signaux représentant un degré de lumière rassemblée ; et
- un système d'examen pour analyser les signaux générés.

15. Le système d'analyse et de préparation d'essai de liquide de la revendication 14 comprenant de plus un moyen de stockage avec des instructions de programme qui sont exécutables par un processeur pour activer de façon sélective l'actionneur ou bien où le système d'analyse et de préparation de l'essai de liquide comprend un cytomètre en flux ou un système optique d'imagerie statique.
